# EUROPÄISCHE PATENTSCHRIFT

(11) **EP 4 458 294 B1**
(45) Veröffentlichungstag und Bekanntmachung des Hinweises auf die Patenterteilung: **02.04.2025**
(21) Anmeldenummer: 23171441.1
(22) Anmeldetag: 04.05.2023
(51) Int. Cl.: A61B 17/88, A61B 34/30, B01F 35/71

(54) **VORRICHTUNG ZUR ABGABE VON KNOCHENZEMENT DURCH OP-ROBOTER**
DEVICE FOR DELIVERING BONE CEMENT BY AN OP-ROBOT
DISPOSITIF DE DISTRIBUTION DE CIMENT OSSEUX PAR ROBOT CHIRURGICAL

(43) Veröffentlichungstag der Anmeldung: 06.11.2024
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- DE-U1- 202004 000 941
- US-A1- 2019 021 796
- US-A1- 2019 254 750

## Beschreibung

### GEBIET DER ERFINDUNG

Die vorliegende Erfindung betrifft das Gebiet der Medizintechnik, insbesondere Vorrichtungen zur Abgabe von Knochenzement durch Operationsroboter (OP-Roboter). Die Vorrichtungen können bei der Implantation von medizinischen Implantaten, wie z.B. Gelenkspacern, verwendet werden. Eine solche Vorrichtung kann mit OP-Robotern verbunden werden oder Bestandteil eines OP-Roboters sein. Weiterhin werden Verfahren zum maschinellen Applizieren von PMMA-Knochenzement beschrieben.

### TECHNISCHER HINTERGRUND

PMMA-Knochenzemente werden in der Chirurgie und Orthopädie zum Fixieren von Gelenkendoprothesen im Knochengewebe verwendet. PMMA-Knochenzemente bestehen üblicherweise aus einer flüssigen Monomerkomponente und einer Pulverkomponente. Die Monomerkomponente enthält üblicherweise das Monomer Methylmethacrylat und einen darin gelösten Aktivator (N,N-Dimethyl-p-toluidin). Die Pulverkomponente, auch als Knochenzementpulver bezeichnet, weist üblicherweise ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs, bis dieser erstarrt. Die oben beschriebenen Ausgangskomponenten, der daraus hergestellte Knochenzementteig und der durch Härtung dieses Knochenzementteigs entstehende Knochenzement werden hierin sowohl einzeln als auch in ihrer Gesamtheit als "Knochenzement" bezeichnet.

Polymethylmethacrylat-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können. Der in den Mischbechern gemischte Knochenzementteig wird üblicherweise mit Spateln appliziert.

Zur Vermeidung von Lufteinschlüssen im Knochenzementteig sind eine Vielzahl von Vakuum-Zementiersystemen beschrieben, bei denen die Zementkomponenten in Kartuschen mit manuell betriebenen Mischern vermischt werden, von denen exemplarisch folgende Dokumente genannt sind: US6,033,105A, US5,624,184A, US4,671,263A, US4,973,168A, US5,100,241A, WO99/67015A1, EP1020167A2, US5,586,821A, EP1016452A2, DE3640279A1, WO94/26403A1, EP1005901A2, US5,344,232A.

Eine Weiterentwicklung in der Zementiertechnik stellen Zementiersysteme dar, in denen sowohl das Zementpulver als auch die Monomerflüssigkeit bereits in separaten Kompartimenten der Kartuschensysteme verpackt sind, und die erst unmittelbar vor der Zementapplikation im Kartuschensystem miteinander vermischt werden. Solche geschlossenen "Full-Prepack-Mischsysteme" sind in den Patenten EP0692229, DE102009031178, US5997544, US6709149, DE69812726 und US5588745 beschrieben.

In den Patentschriften DE102016121606B4 und DE102016121607B4 wurde ein Full-Prepacked-Mischsystem beschrieben. Bei diesem Mischsystem wird die Kartusche senkrecht mit dem Kartuschenkopf vertikal nach oben gehalten und die Monomerflüssigkeit wird mit einer Auspressvorrichtung von unten in verdichtetes Zementpulver eingepresst, wobei das Zementpulver durch die Monomerflüssigkeit benetzt wird und die zwischen den Zementpulverpartikeln befindliche Luft durch die Monomerflüssigkeit nach oben herausgepresst wird. Es entsteht ein blasenfreier Zementteig ohne Einwirkung von mechanischen Mischvorrichtungen. Wie unten ausführlicher dargestellt ist, können solche Full-Prepack-Mischsysteme in besonders vorteilhafter Weise gemeinsam mit den hierin beschriebenen Vorrichtungen eingesetzt werden.

US2019021796A1 beschreibt Methoden und Systeme für die roboterassistierte Chirurgie. Ein darin offenbartes Robotersystem umfasst einen Lokalisierer, einen chirurgischen Robotermanipulator, einen Endeffektor, der so konfiguriert ist, dass er abnehmbar mit dem Manipulator verbunden werden kann, und eine Steuerung. Der Controller ist dazu konfiguriert, Signale vom Lokalisierer zu empfangen, eine Endposition eines zum Durchdringen eines Teils der Anatomie eines Patienten verwendeten Werkzeugs zur Schaffung einer Kavität basierend auf den vom Lokalisierer empfangenen Signalen zu bestimmen und einen Implantateinführungspfad für ein Implantat zu bestimmen, das in eine endgültige Implantatposition innerhalb des Teils der Anatomie des Patienten eingesetzt werden soll, wobei die endgültige Implantatposition der Endposition des Werkzeugs zur Schaffung einer Kavität entspricht. Der Controller ist außerdem so konfiguriert, dass er den Endeffektor mit dem daran gekoppelten Implantat so bewegt, dass sich das Implantat entlang des Implantateinführpfads bewegt, und die Bewegung des Endeffektors beendet, wenn das Implantat die endgültige Implantatposition erreicht.

### BEVORZUGTE AUSFÜHRUNGSFORMEN

Bei den bisher bekannten Vakuummischsystemen und den Prepacked-Mischsystemen wird der PMMA-Zementteig häufig derart appliziert, dass eine mit PMMA-Zementteig gefüllte Kartusche mit einer manuell bedienbaren Auspressvorrichtung verbunden wird und anschließend der Zementteig manuell mit dieser Auspressvorrichtung aus der Kartusche ausgepresst wird. Dies kann beispielsweise im Rahmen der Implantation einer Kniegelenkendoprothese erfolgen. Der medizinische Anwender kann dabei während der Zementauspressung das Austragsrohr über die zu zementierenden Flächen der Kniegelenkendoprothese und gegebenenfalls über das knöcherne Implantatlager der Tibia und des Femurs bewegen. Dadurch kann der Zementteig gleichmäßig verteilt werden, und die Tibia- und die Femurkomponente der Kniegelenkendoprothese können anschließend auf die zuvor präparierte Tibia und den präparierten Femur aufgepresst werden. Hierdurch können eine gleichmäßige Zementverteilung und ein homogener Verbund zwischen dem Knochengewebe und den Gelenkkomponenten ermöglicht werden. Der überschüssige Zementteig kann manuell entfernt werden. Bei der Zementierung von Hüftgelenkendoprothesen kann die Zementierung des Acetabulum-Cups in ähnlicher Weise erfolgen. Die Zementierung des Schaftes von Hüftgelenkendoprothesen wird so vorgenommen, dass der zuvor präparierte Kanal im proximalen Femur retrograd mit Zementteig gefüllt wird. Es erfolgt dann mit einem Pressurizer ein Einpressen des Zementteigs in die Spongiosa. Danach wird der Schaft der Hüftprothese in den mit Knochenzementteig befüllten Kanal des Femurs eingesteckt unter Verdrängung des überschüssigen Knochenzementteigs.

Zunehmend werden in der Chirurgie und Orthopädie OP-Roboter eingesetzt. Diese spielen insbesondere bei der Implantation von zementfreien Kniegelenkendoprothesen eine Rolle. Es wäre wünschenswert, wenn beispielsweise auch zementierte Prothesen, zum Beispiel Kniegelenk- und Hüftgelenkendoprothesen, mit Hilfe von OP-Robotern implantiert werden könnten.

Die Aufgabe der Erfindung besteht in der Bereitstellung einer Vorrichtung zum maschinellen Applizieren von Knochenzement, beispielsweise PMMA-Knochenzement, die mit einem OP-Roboter verbunden werden kann oder verbunden ist. Die Vorrichtung kann es ermöglichen, zuvor vom Operateur definierte Knochenzementvolumina aus Kartuschen maschinell in einem vorbestimmten Zeitraum zu applizieren. Während des Zementiervorgangs kann die Vorrichtung bevorzugt durch einen Roboterarm bewegt werden. Die Vorrichtung kann bevorzugt in der Lage sein, eine genaue Dosierung der auszupressenden Zementvolumina vorzunehmen. Diese Dosierung kann bevorzugt zeitlich steuerbar sein, damit ein OP-Roboter durch definierte Bewegung in den Richtungen der x-Achse, der y-Achse und der z-Achse den Knochenzementteig exakt auf dem Implantat und auf dem Knochengewebe positionieren kann. Dazu kann die Vorrichtung so beschaffen sein, dass diese mit Kartuschen verbunden werden kann, die bereits zuvor gemischten PMMA-Zementteig enthalten. Das bedeutet, dass der medizinische Anwender die Zementkomponenten in einer Kartusche zu einem PMMA-Knochenzementteig vermischt und anschließend die Kartusche mit der Vorrichtung verbindet, die sich bereits am Roboterarm befindet oder mit dem Roboterarm verbunden werden kann. In einigen Ausführungsformen kann die Vorrichtung mit einer Kartusche verbunden werden, welche die ungemischten Zementkomponenten, beispielsweise eine Monomerflüssigkeit und ein Zementpulver, enthält. Durch eine hierin beschriebene Vorrichtung können in diesem Fall die Zementkomponenten durch eine Bewegung des Austragskolbens in Richtung des Kartuschenkopfs gemäß den Patentschriften DE102016121606B4 und DE102016121607B4 vermischt und anschließend nach Bildung des Knochenzementteigs aus der Kartusche ausgepresst werden. Die hierin beschriebene Vorrichtung kann in einer Variante so gestaltet sein, dass die Verwendung von Elektromotoren und komplexen Getrieben zum Auspressen des Knochenzementteigs entbehrlich ist. Hierzu können beispielsweise pneumatische oder hydraulische Antriebe vorgesehen sein. Die Quelle des pneumatischen oder hydraulischen Drucks kann bevorzugt außerhalb der hierin beschriebenen Vorrichtung und außerhalb des verbundenen Roboters angeordnet sein. Dadurch kann die Gewichtsbelastung der Roboterarme begrenzt werden, und es kann eine kostengünstige Fertigung der Vorrichtung ermöglicht werden. In einigen Ausführungsformen kann die hierin beschriebene Vorrichtung eingerichtet sein, den Auspressvorgang des Knochenzements unter Verwendung von Elektromotoren und Getrieben durchzuführen. Weiterhin können in einigen Ausführungsformen zum Antrieb im OP bereits vorhandene, gut steuerbare Energiequellen zum Antrieb der hierin beschriebenen Vorrichtung genutzt werden.

Die Aufgabe der vorliegenden Erfindung besteht darin, eines oder mehrere der oben geschilderten und weitere Probleme des Stands der Technik zu lösen. Die Erfindung ermöglicht eine maschinelle Abgabe von Knochenzement mithilfe eines OP-Roboters.

Diese Aufgabe wird gelöst durch die hierin beschriebenen Vorrichtungen, die in den Patentansprüchen beschrieben sind.

Bevorzugte Ausführungsformen der Erfindung werden nachstehend beschrieben.

Eine erste Ausführungsform beschreibt eine Vorrichtung zur maschinellen Abgabe von Knochenzement, aufweisend eine Verbindungseinheit, die zur Verbindung mit einem chirurgischen Roboter eingerichtet ist, eine Auspresseinheit, die zur Abgabe von Knochenzement eingerichtet ist, eine Aufnahmeeinheit, die zur Aufnahme eines Behälters mit Knochenzement eingerichtet ist, und eine Schnittstelle, die zur Verbindung mit einer Steuereinheit eingerichtet ist, um mit Hilfe der Auspresseinheit eine vorbestimmte Menge Knochenzement aus dem Behälter abzugeben, wobei die Vorrichtung weiterhin einen Sensor aufweist, welcher operativ mit der Schnittstelle verbunden ist, um die Menge des mithilfe der Auspresseinheit abgegebenen Knochenzements zu bestimmen.

Eine zweite Ausführungsform beschreibt eine Vorrichtung gemäß der ersten Ausführungsform, wobei die Schnittstelle eingerichtet ist, die Menge des mithilfe der Auspresseinheit abgegebenen Knochenzements zu übermitteln und bei Erreichen der vorbestimmten Menge eine weitere Abgabe des Knochenzements zu stoppen.

Eine vierte Ausführungsform beschreibt eine Vorrichtung nach einer der vorangehenden Ausführungsformen, wobei der Sensor ein optischer Sensor, ein mechanischer Sensor oder ein magnetischer Sensor ist.

Eine fünfte Ausführungsform beschreibt eine Vorrichtung gemäß der dritten oder vierten Ausführungsform, wobei der Sensor eingerichtet ist, eine räumliche Position einer Markierung zu detektieren, um dadurch die Menge des mithilfe der Auspresseinheit abgegebenen Knochenzements zu bestimmen, wobei die Markierung bevorzugt eine farbiges Bauteil der Auspresseinheit, besonders bevorzugt ein Dichtungsring eines Kolbens, ist.

Eine sechste Ausführungsform beschreibt eine Vorrichtung gemäß einer der dritten bis fünften Ausführungsform, wobei der Sensor und/oder die Schnittstelle eingerichtet ist/sind, ein inneres Volumen der Auspresseinheit und/oder des Behälters zu ermitteln.

Eine siebte Ausführungsform beschreibt eine Vorrichtung nach einer der dritten bis sechsten Ausführungsform, wobei der Sensor auf einer Sensorleiste angeordnet ist, die sich auf einer Außenseite der Vorrichtung entlang der Aufnahmeeinheit erstreckt.

Eine achte Ausführungsform beschreibt eine Vorrichtung nach einer der vorangehenden Ausführungsformen, wobei die Verbindungseinheit einen elektrischen Anschluss aufweist, welcher zur Energieversorgung der Vorrichtung und/oder zum Empfang eines Steuersignals eingerichtet ist.

Eine neunte Ausführungsform beschreibt eine Vorrichtung nach einer der vorangehenden Ausführungsformen, wobei die Verbindungseinheit einen Anschluss zur Verbindung mit einem druckbeaufschlagten Medium aufweist, wobei das druckbeaufschlagte Medium bevorzugt Druckluft, eine Vakuumquelle oder ein hydraulisches Medium ist.

Eine zehnte Ausführungsform beschreibt eine Vorrichtung nach einer der vorangehenden Ausführungsformen, wobei die Auspresseinheit einen Antrieb aufweist, der zur Abgabe von Knochenzement mit Hilfe der Auspresseinheit eingerichtet ist.

Eine elfte Ausführungsform beschreibt eine Vorrichtung gemäß der zehnten Ausführungsform, wobei der Antrieb ein hydraulischer, pneumatischer oder elektrischer Antrieb ist.

Eine zwölfte Ausführungsform beschreibt eine Vorrichtung gemäß der zehnten oder elften Ausführungsform, wobei die Schnittstelle zur Steuerung des Antriebs eingerichtet ist.

Eine dreizehnte Ausführungsform beschreibt eine Vorrichtung nach einer der vorangehenden Ausführungsformen, wobei die Vorrichtung weiterhin zum maschinellen Mischen von Knochenzement in dem Behälter eingerichtet ist.

Weiterhin betrifft die Erfindung ein Computerprogramm zur Steuerung der Vorrichtung nach einer der vorangehenden Ausführungsformen, wobei das Computerprogramm zum Betrieb der Auspresseinheit und/oder des Sensors eingerichtet ist.

Weiterhin betrifft die Erfindung ein Kit, aufweisend eine Vorrichtung gemäß einer der ersten bis dreizehnten Ausführungsformen, und einen Behälter mit Knochenzement, wobei der Behälter bevorzugt zum Mischen von Knochenzement eingerichtet ist.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Die Erfindung betrifft in einer ersten Ausführungsform eine Vorrichtung zur maschinellen Abgabe von Knochenzement, aufweisend eine Verbindungseinheit, die zur Verbindung mit einem chirurgischen Roboter eingerichtet ist, eine Auspresseinheit, die zur Abgabe von Knochenzement eingerichtet ist, eine Aufnahmeeinheit, die zur Aufnahme eines Behälters mit Knochenzement eingerichtet ist, und eine Schnittstelle, die zur Verbindung mit einer Steuereinheit eingerichtet ist, um mit Hilfe der Auspresseinheit eine vorbestimmte Menge Knochenzement aus dem Behälter abzugeben, wobei die Vorrichtung weiterhin einen Sensor aufweist, welcher operativ mit der Schnittstelle verbunden ist, um die Menge des mithilfe der Auspresseinheit abgegebenen Knochenzements zu bestimmen.

Die hierin beschriebenen Vorrichtungen sind zur maschinellen Abgabe von Knochenzement vorgesehen. Demnach sind diese Vorrichtungen bevorzugt dazu eingerichtet, Knochenzement mithilfe eines Antriebs abzugeben, welcher elektronisch gesteuert wird. Die Vorrichtungen können in vorteilhafter Weise in Verbindung mit chirurgischen Robotern verwendet werden, welche hierin auch als OP-Roboter bezeichnet werden. Ein Beispiel eines chirurgischen Roboters ist in EP3743004A1 beschrieben. Solche Roboter umfassen bevorzugt eine Antriebseinheit und eine Steuereinheit, um den Betrieb des Roboters zu ermöglichen. Die hierin beschriebenen Vorrichtungen umfassen eine Schnittstelle, welche eingerichtet ist, mit einer solchen Steuereinheit eines Roboters verbunden zu werden. Hierdurch kann die erfindungsgemäße Vorrichtung operativ mit der Steuereinheit des Roboters zusammenwirken. Beispielsweise kann die Steuereinheit des Roboters die Abgabe von Knochenzement aus der erfindungsgemäßen Vorrichtung mithilfe der Schnittstelle steuern. Die Schnittstelle kann dazu mit einer Auspresseinheit der Vorrichtung operativ verbunden sein. In einigen Ausführungsformen ist die Schnittstelle eingerichtet, ein Steuersignal aus der Steuereinheit eines Roboters an die Auspresseinheit weiterzugeben. In einigen Ausführungsformen kann die Schnittstelle eingerichtet sein, ein solches Steuersignal aus der Steuereinheit zu verarbeiten, und das verarbeitete Steuersignal an die Auspresseinheit abzugeben.

In einigen Ausführungsformen kann die Vorrichtung an einem Roboterarm eines OP-Roboters angebracht werden. Hierdurch kann es ermöglicht werden, durch Zusammenwirkung des Roboterarms mit der Vorrichtung die räumliche Positionierung des aus der Vorrichtung abgegebenen Knochenzements zu steuern. Bevorzugt kann hierdurch eine dreidimensional steuerbare Abgabe von Knochenzement erfolgen.

Die hierin beschriebenen Ausgangskomponenten eines Knochenzements, zum Beispiel eine Pulverkomponente und eine flüssige Komponente, der daraus hergestellte Knochenzementteig, und der durch Härtung dieses Knochenzementteigs entstehende Knochenzement werden hierin sowohl einzeln als auch in ihrer Gesamtheit als "Knochenzement" bezeichnet. Ein bevorzugtes Beispiel für einen Knochenzement ist ein PMMA-Knochenzement. Die Vorrichtung kann entweder eingerichtet sein, einen Behälter aufzunehmen, welcher bereits fertig angemischten Knochenzement enthält, oder einen Behälter aufzunehmen, welcher mehrere getrennte Komponenten eines Knochenzements enthält. Die Vorrichtung kann zum Mischen dieser Komponenten eingerichtet sein. Dieser Mischvorgang kann bevorzugt durch einen OP-Roboter steuerbar sein.

Die erfindungsgemäße Vorrichtung umfasst eine Aufnahmeeinheit, welche zur Aufnahme eines Behälters mit Knochenzement eingerichtet ist. In einigen Ausführungsformen ist die Aufnahmeeinheit eingerichtet, handelsübliche Austragsvorrichtungen oder Mischvorrichtungen für Knochenzement aufzunehmen. In einigen Ausführungsformen ist die Aufnahmeeinheit eingerichtet, einen Behälter aufzunehmen, welcher die benötigten Komponenten zur Herstellung eines Knochenzements in voneinander getrennten Kompartimenten enthält. In einer Ausführungsform ist die Aufnahmeeinheit eingerichtet, einen Behälter aufzunehmen, in welchem Knochenzement gemischt und anschließend ausgetragen werden kann. In einigen Ausführungsformen ist die Vorrichtung eingerichtet, mithilfe eines Roboters Knochenzement in einem aufzunehmenden Behälter zu mischen, und mithilfe der Auspresseinheit auszutragen. Besonders bevorzugte Behälter, mit denen die erfindungsgemäße Vorrichtung verwendet werden kann, sind in DE102016121606B4 und DE102016121607B4 beschrieben, die hiermit durch Bezugnahme vollständig mitaufgenommen sind. Die DE102016121606B4 beschreibt einen Knochenzementapplikator zum Mischen und Applizieren eines Knochenzements, bei dem die Ausgangskomponenten des Knochenzements in einer geschlossenen Kartusche zu einem Knochenzementteig mischbar sind, wobei die Kartusche ein mehrteiliges Verschlusssystem umfassend eine Austragsöffnung aufweist, wobei zumindest zwei Teile des Verschlusssystems angetrieben durch eine Bewegung des gemischten Knochenzementteigs gegeneinander bewegbar sind und sich durch diese Bewegung der zumindest zwei Teile des Verschlusssystems gegeneinander die Austragsöffnung öffnet, und wobei die Bewegung des gemischten Knochenzementteigs durch einen Druck auf den Knochenzementteig anzutreiben ist.

Zur Aufnahme eines Behälters mit Knochenzement kann die Auspresseinheit mit einem geeigneten Verbindungselement versehen sein, die beispielsweise für eine formschlüssige Verbindung mit dem Behälter eingerichtet ist. Beispielsweise kann es sich bei einem solchen Verbindungselement um einen Bajonettverschluss oder einen Schnappverschluss handeln. Das Verbindungselement kann Haken, Laschen oder Gewinde aufweisen, die eine formschlüssige Verbindung mit dem Behälter ermöglichen.

Die Vorrichtung umfasst eine Auspresseinheit, die zur Abgabe von Knochenzement eingerichtet ist. Die Auspresseinheit kann beispielsweise um einen Kolben aufweisen, welcher eingerichtet ist, Druck auf einen aufgenommenen Behälter mit Knochenzement auszuüben. Die Auspresseinheit kann einen beweglichen Teller aufweisen, welcher eingerichtet ist, Druck auf einen aufgenommenen Behälter mit Knochenzement auszuüben. Der Kolben oder Teller kann angeordnet sein, um sich axial in einen aufgenommenen Behälter mit Knochenzement zu bewegen.

Die Vorrichtung kann ein Gehäuse umfassen, welches beispielsweise eine im wesentlichen zylindrische Grundform aufweisen kann. Die Auspresseinheit kann innerhalb dieses Gehäuses angeordnet sein. Am Gehäuse kann ein Widerlager für die Auspresseinheit angeordnet und befestigt sein. Das Widerlager kann beispielsweise mithilfe einer Schraube oder Niete an dem Gehäuse befestigt sein.

In einer Ausführungsform ist die Vorrichtung eingerichtet, die Menge des mithilfe der Auspresseinheit abgegebenen Knochenzements zu bestimmen. Dies kann beispielsweise durch Bestimmung der Position eines Elements der Auspresseinheit erfolgen, beispielsweise der Position eines Kolbens oder Tellers, welche Druck auf den Behälter mit Knochenzement ausüben, um Knochenzement aus dem Behälter abzugeben. Das Element der Auspresseinheit kann hierzu eine Markierung aufweisen, deren Position mit einem Sensor bestimmbar ist.

In einer Ausführungsform ist die Schnittstelle eingerichtet, die Menge des mithilfe der Auspresseinheit abgegebenen Knochenzements an den Roboter zu übermitteln und bei Erreichen der vorbestimmten Menge eine weitere Abgabe des Knochenzements zu stoppen.

Erfindungsgemäß weist die Vorrichtung einen Sensor auf. Der Sensor ist operativ mit der Schnittstelle verbunden, um die Menge des mithilfe der Auspresseinheit abgegebenen Knochenzements zu bestimmen und gegebenenfalls an den Roboter zu übermitteln.

Der Sensor kann ein optischer Sensor, ein mechanischer Sensor oder ein magnetischer Sensor sein. Der optische Sensor kann beispielsweise eine Kamera oder eine Photodiode sein. Die Vorrichtung kann weiterhin eine Lichtquelle umfassen, zum Beispiel eine Leuchtdiode oder ein Laser. Der Sensor kann beispielsweise ein Sensor mit Laufzeitmessung, ein Sensor mit Phasenmodulation, ein Triangulationssensor, oder ein interferometrischer Sensor sein. Der magnetischer Sensor kann beispielsweise eingerichtet sein, nach dem Funktionsprinzip der Magnetostriktion eine relative Position eines beweglichen Teils der Auspresseinheit zu ermitteln. Ein solcher Sensor kann beispielsweise eine innerhalb der Vorrichtung unbeweglich angeordnete Messbasis, einen Wellenleiter, einen beweglichen Magneten und einen Signalwandler enthalten. Hierbei kann durch eine magnetische Kraft eine mechanische Schwingung in dem Wellenleiter erzeugt werden. Durch Bestimmung der Laufzeit dieser Schwingung können Entfernungen zwischen zwei definierten Punkten gemessen werden. Der Signalwandler kann eingerichtet sein, eine mechanische Schwingung des Sensors in ein Messsignal umzuwandeln.

Der Sensor kann eingerichtet sein, den Verfahrweg eines Elements der Auspresseinheit zu detektieren. Beispielsweise kann dies durch operative Zusammenwirkung des Sensors mit einem Antrieb der Auspresseinheit erfolgen.

In einer Ausführungsform ist der Sensor eingerichtet, eine räumliche Position einer Markierung zu detektieren, um dadurch die Menge des mithilfe der Auspresseinheit abgegebenen Knochenzements zu bestimmen. Die Markierung kann bevorzugt ein farbiges Bauteil der Auspresseinheit sein, zum Beispiel ein Dichtungsring eines Kolbens oder Tellers. Die Markierung kann sich farblich von den sie umgebenden Elementen der Vorrichtung unterscheiden.

In einer Ausführungsform ist der Sensor eingerichtet, ein inneres Volumen der Auspresseinheit zu ermitteln. In einer Ausführungsform ist der Sensor eingerichtet, ein inneres Volumen des Behälters mit Knochenzement zu ermitteln. In einer Ausführungsform ist die Schnittstelle eingerichtet, eine inneres Volumen der Auspresseinheit zu ermitteln. In einer Ausführungsform ist die Schnittstelle eingerichtet, ein inneres Volumen des Behälters mit Knochenzement zu ermitteln.

Auf diese Weise kann die Menge des mithilfe der Vorrichtung abgegebenen Knochenzements ermittelt werden.

Der Sensor kann auf einer Sensorleiste angeordnet sein. Die Sensorleiste kann in zweckmäßiger Weise an der Vorrichtung angebracht sein, um die Menge des mithilfe der Auspresseinheit abgegebenen Knochenzements zu bestimmen. In einer Ausführungsform erstreckt sich die Sensorleiste auf einer Außenseite der Vorrichtung entlang der Aufnahmeeinheit oder entlang der Auspresseinheit. Die Sensorleiste kann sich in axialer oder longitudinaler Richtung entlang der Vorrichtung erstrecken. Gegebenenfalls können mehrere Sensoren vorgesehen sein. Die Sensoren können beispielsweise umlaufend entlang der Gehäuseaußenseite der Vorrichtung angeordnet sein.

In einer weiteren Ausführungsform weist die Verbindungseinheit einen elektrischen Anschluss auf. Der elektrische Anschluss kann zur Energieversorgung der Vorrichtung eingerichtet sein. Insbesondere kann der elektrische Anschluss zur Energieversorgung der Auspresseinheit und/oder des Sensors eingerichtet sein. Der elektrische Anschluss kann alternativ oder zusätzlich zum Empfang eines Steuersignals eingerichtet sein. Solche Steuersignale können beispielsweise der externen Ansteuerung der verschiedenen Teile der Vorrichtung durch einen OP-Roboter oder durch eine andere externe Steuervorrichtung dienen. Beispielsweise können der Sensor und/oder die Auspresseinheit auf diese Weise extern gesteuert und/oder überwacht werden.

In einer Ausführungsform weist die Verbindungseinheit einen Anschluss zur Verbindung mit einem druckbeaufschlagten Medium auf. Das druckbeaufschlagte Medium kann beispielsweise Druckluft, eine Vakuumquelle oder ein hydraulisches Medium sein. Das druckbeaufschlagte Medium weist einen Druck auf, der deutlich höher oder deutlich niedriger als der übliche Umgebungsdruck von ungefähr 1000 hPa ist. Das druckbeaufschlagte Medium kann den Antrieb der Auspresseinheit dienen. Demnach ist die Auspresseinheit in einer Ausführungsform dazu eingerichtet, durch ein druckbeaufschlagtes Medium angetrieben zu werden.

In einer Ausführungsform weist die Auspresseinheit einen Antrieb auf, der zur Abgabe von Knochenzement mit Hilfe der Auspresseinheit eingerichtet ist. Hierzu kann der Antrieb operativ mit der Auspresseinheit verbunden sein, sodass die Auspresseinheit durch Einwirkung auf einen Behälter mit Knochenzement die Abgabe von Knochenzement bewirken kann.

In einer Ausführungsform ist der Antrieb ein hydraulischer, pneumatischer oder elektrischer Antrieb. Ein hydraulischer oder pneumatischer Antrieb kann fluidleitend mit einem oben beschriebenen Anschluss zur Verbindung mit einem druckbeaufschlagten Medium verbunden sein. Die Vorrichtung kann demnach einen Eingang und einen Ausgang für ein druckbeaufschlagtes Medium aufweisen. Der Eingang und/oder der Ausgang können eine mechanische oder elektrische Steuerung umfassen. Der Eingang und/oder der Ausgang können jeweils ein Ventil umfassen. Insbesondere wenn die Vorrichtung einen pneumatischen Antrieb umfasst, kann der Ausgang weiterhin einen Filter umfassen, um das abgegebene Fluidmedium, zum Beispiel Druckluft, zu reinigen. Besonders vorteilhaft sind Filter, welche eine Kontamination der Umgebungsluft mit Keimen verhindern können. Solche Filter werden im Fachgebiet auch als Sterilfilter bezeichnet. Solche Filter können beispielsweise eine Porengröße von 50 bis 500 nm aufweisen, wobei sich die Porengröße auf das nominelle Volumen der zurückgehaltenen Partikel bezieht.

In einer Ausführungsform ist die Schnittstelle zur Steuerung des Antriebs eingerichtet. Beispielsweise kann die Schnittstelle ein externes Steuersignal an die Vorrichtung weiterleiten, um einen Elektromotor oder ein Steuerungsventil eines hydraulischen oder pneumatischen Antriebs zu steuern, um eine vorbestimmte Menge Knochenzement mithilfe der Vorrichtung abzugeben.

Die Vorrichtung umfasst eine Aufnahmeeinheit, die zur Aufnahme eines Behälters mit Knochenzement eingerichtet ist. Der Behälter kann entweder fertig angemischten Knochenzement enthalten, oder kann getrennt einzelne Komponenten eines Knochenzements enthalten. In einer Ausführungsform ist die Vorrichtung zum maschinellen Mischen von Knochenzement in dem Behälter eingerichtet. In diesem Fall muss der Knochenzement nicht durch das OP-Personal gemischt werden, sondern die Vorrichtung übernimmt den Mischvorgang, gegebenenfalls gesteuert durch einen OP-Roboter, welcher die Vorrichtung entsprechend steuert. Somit kann gemäß einer solchen Ausführungsform mithilfe der Vorrichtung der Knochenzement durch einen OP-Roboter sowohl gemischt als auch abgegeben werden. In einer Ausführungsform ist die Vorrichtung eingerichtet, den Knochenzement mithilfe eines OP-Roboters gezielt an eine oder mehrere vorgegebene räumliche Positionen abzugeben. Hierdurch kann für den behandelnden Chirurgen die manuelle Führung einer Knochenzement-Austragsvorrichtung entfallen, da diese Aufgabe durch den OP-Roboter mithilfe der hierin beschriebenen Vorrichtung übernommen wird.

Die Erfindung betrifft auch ein Computerprogramm zur Steuerung der hierin beschriebenen Vorrichtung. Das Computerprogramm kann insbesondere zum Betrieb der Auspresseinheit eingerichtet sein. Das Computerprogramm kann alternativ oder zusätzlich zum Betrieb des Sensors eingerichtet sein. Das Computerprogramm kann auf einem Datenträger gespeichert sein. Demnach umfasst eine Ausführungsform auch einen Datenträger, auf dem ein solches Computerprogramm gespeichert ist. Das Computerprogramm kann beispielsweise einen oder mehrere oder alle der folgenden Schritte aufweisen, bzw. zur Durchführung dieser Schritte eingerichtet sein:
1) Aktivieren der Auspresseinheit, wobei durch Aktivieren der Auspresseinheit die Abgabe von Knochenzement mithilfe der Vorrichtung erfolgt;
2) Berechnung der Menge des mithilfe der Vorrichtung abgegebenen Knochenzements, basierend auf einem Messwert eines Sensors der Vorrichtung
3) Vergleich dieser Menge mit einem vorbestimmten Wert;
4) Deaktivieren der Auspresseinheit, wenn die Menge den vorbestimmten Wert erreicht oder überschreitet.

Die Erfindung betrifft auch ein Kit, aufweisend eine hierin beschriebene Vorrichtung, und einen Behälter mit Knochenzement, wobei der Behälter bevorzugt zum Mischen und/oder zur Abgabe von Knochenzement eingerichtet ist.

Der Behälter kann ein Full-Prepack-Mischsystem sein. Full-Prepack-Mischsysteme stellen Zementiersysteme dar, in denen sowohl das Zementpulver als auch die Monomerflüssigkeit in separaten Kompartimenten der Kartuschensysteme verpackt sind, um erst unmittelbar vor der Zementapplikation im Kartuschensystem miteinander zu Knochenzement vermischt zu werden. Beispiele für Full-Prepack-Mischsysteme sind in DE102016121606B4 und DE102016121607B4 beschrieben. Bei einem solchen Mischsystem kann die Kartusche senkrecht mit dem Kartuschenkof vertikal nach oben gehalten werden und die Monomerflüssigkeit mit einer Auspressvorrichtung von unten in verdichtetes Zementpulver eingepresst werden, wobei das Zementpulver durch die Monomerflüssigkeit benetzt werden kann und die zwischen den Zementpulverpartikeln befindliche Luft durch die Monomerflüssigkeit nach oben herausgepresst werden kann.

Der Behälter enthält bevorzugt ein Verbindungselement, beispielsweise ein formschlüssiges Verbindungselement, welches zur Verbindung mit einer hierin beschriebenen Vorrichtung eingerichtet ist. Der Behälter kann weiterhin einen Mischstab aufweisen. Das Kit kann weiterhin einen Schnorchel zum Ausbringen von Knochenzement aus dem Behälter aufweisen. Der Knochenzement kann ein Antibiotikum aufweisen.

Weiterhin wird ein OP-Roboter beschrieben (nicht beansprucht), welcher zur Abgabe von Knochenzement mithilfe einer hierin beschriebenen Vorrichtung, eines hierin beschriebenen Computerprogramms und/oder eines hierin beschriebenen Kits eingerichtet ist. Der OP-Roboter kann eine hierin beschriebene Vorrichtung als integralen Bestandteil enthalten, oder kann lediglich zur Verbindung mit einer solchen Vorrichtung eingerichtet sein. Beispielsweise kann der OP-Roboter Anschlüsse für das Verbindungselement der Vorrichtung aufweisen. Der Roboter kann zur Überwachung des Sensors der Vorrichtung und/oder zur Steuerung der Auspressvorrichtung der Vorrichtung eingerichtet sein. Der Roboter kann zur operativen Verbindung an einen hierin beschriebenen elektrischen Anschluss der Vorrichtung eingerichtet sein. Der Roboter kann weiterhin Anschlüsse zur Versorgung der Vorrichtung mit einem druckbeaufschlagten Medium aufweisen, welches zum Antrieb der Auspressvorrichtung dienen kann. Der OP-Roboter kann zum Mischen von Knochenzement und/oder zur Abgabe von Knochenzement mithilfe einer hierin beschriebenen Vorrichtung ausgebildet und eingerichtet sein.

Der OP-Roboter kann weiterhin ein Computerprogramm zur Steuerung einer hierin beschriebenen Vorrichtung enthalten, wie in den Patentansprüchen beschrieben. Hierzu wird auf die vorangegangenen Ausführungen hierin verwiesen.

Weiterhin wird ein therapeutisches Behandlungsverfahren beschrieben (nicht beansprucht), welches die Abgabe von Knochenzement mithilfe einer hierin beschriebenen Vorrichtung, eines hierin beschriebenen Computerprogramms, eines hierin beschriebenen OP-Roboters und/oder eines hierin beschriebenen Kits umfasst.

### BEISPIELE

Die Erfindung wird nachfolgend anhand von Beispielen weiter verdeutlicht.

### FIGUREN

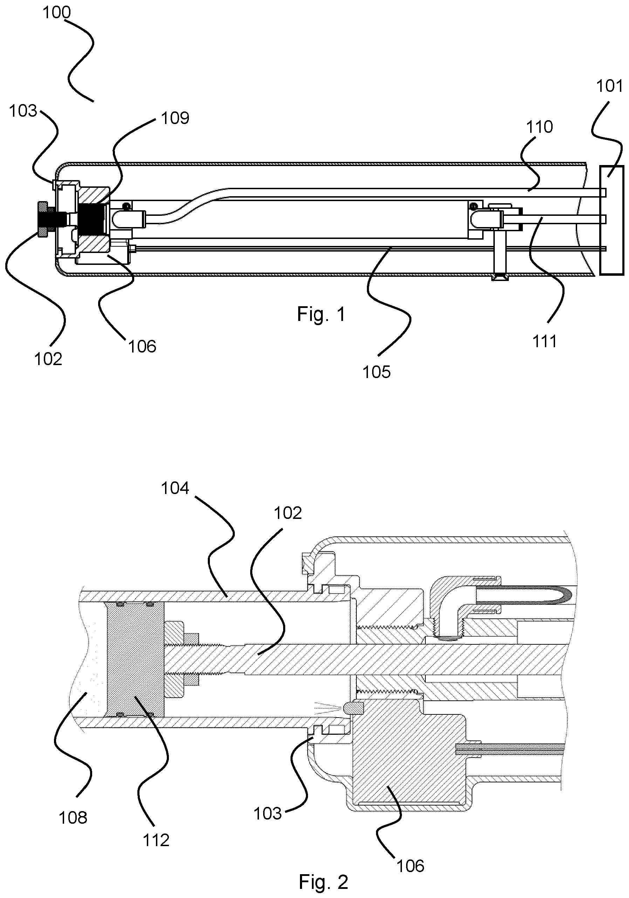

**Fig. 1** zeigt beispielhaft eine Ausführungsform einer erfindungsgemäßen Vorrichtung **100.** Die Vorrichtung **100** weist eine Verbindungseinheit **101** auf, die zur Verbindung mit einem OP-Roboter **200** eingerichtet ist. Im hier gezeigten Beispiel umfasst die Verbindungseinheit **101** auch einen Eingang **110** und einen Ausgang **111,** welche jeweils zum Anschluss an ein externes druckbeaufschlagtes Medium dienen. An den Eingang **110** kann beispielsweise eine Druckluftquelle angeschlossen werden, die zum Antrieb der Auspresseinheit **102** der Vorrichtung dienen kann. Die verwendete Druckluft wird anschließend durch den Ausgang **111** wieder aus der Vorrichtung herausgeführt. Der Ausgang **111** kann einen Filter aufweisen, um die austretende Luft zu reinigen. Die Verbindungseinheit **101** umfasst weiterhin eine Schnittstelle **105,** welche zur Steuerung der Auspresseinheit **102** eingerichtet ist. Beispielsweise kann ein OP-Roboter, der über die Verbindungseinheit **101** operativ mit der Vorrichtung **100** verbunden ist, über die Schnittstelle **105** die Menge des mithilfe der Auspresseinheit **102** abgegebenen Knochenzements steuern. Die Vorrichtung umfasst weiterhin einen Sensor **106,** um die Menge des mithilfe der Auspresseinheit **102** abgegebenen Knochenzements zu erfassen. Der Sensor **106** ist über die Schnittstelle **105** mit einer externen Steuerung verbunden, welche Teil eines OP-Roboters **200** sein kann. Die Auspresseinheit **102** wird mithilfe eines Antriebs **109** bewegt, um Knochenzement aus der Vorrichtung **100** abzugeben. Weiterhin enthält die Vorrichtung eine Aufnahmeeinheit **103,** die zur Aufnahme eines Behälters **104** mit Knochenzement eingerichtet ist. Die Aufnahmeeinheit kann dazu ein formschlüssiges Verbindungselement, beispielsweise einen Bajonettverschluss oder ein Gewinde, aufweisen.
**Fig. 2** zeigt eine Querschnittsansicht einer erfindungsgemäßen Vorrichtung, welche über die Aufnahmeeinheit **103** mit einem Behälter **104** mit Knochenzement verbunden ist. Der Knochenzement ist in einem inneren Volumen **108** des Behälters **104** angeordnet. Die Auspresseinheit **102** weist einen Teller **112** auf, der innerhalb des Behälters **104** beweglich angeordnet ist, und eingerichtet ist, den Knochenzement aus dem inneren Volumen **108** auszubringen. Die Position des Tellers **112** wird mithilfe eines Sensors **106** überwacht, worüber die Menge des ausgebrachten Knochenzements bestimmt werden kann.

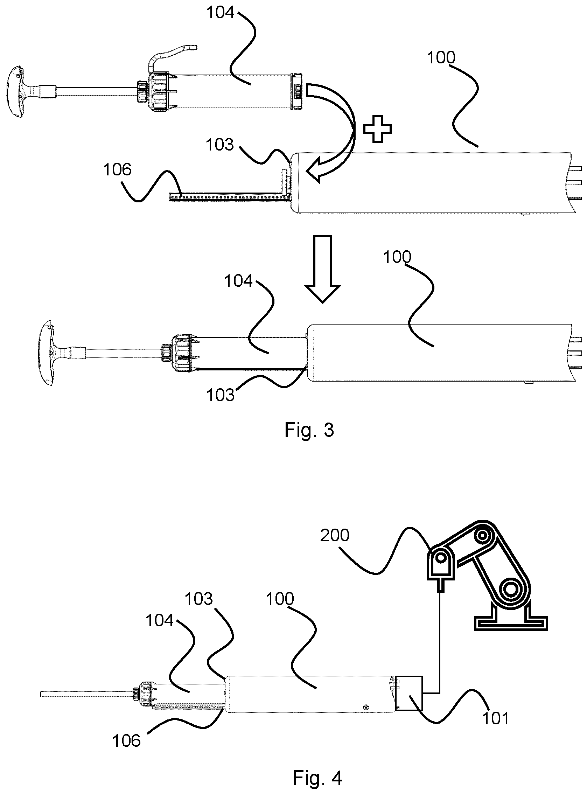

**Fig. 3** zeigt eine weitere Ausführungsform einer erfindungsgemäßen Vorrichtung **100.** Eine handelsübliche Kartusche **104** mit Knochenzement wird an die Aufnahmeeinheit **103** der Vorrichtung angebracht. Die Vorrichtung umfasst einen Sensor **106,** die in diesem Beispiel als Sensorleiste ausgestaltet ist, welche mehrere in Reihe angeordnete einzelne Sensorelemente umfasst.
**Fig. 4** zeigt eine erfindungsgemäße Vorrichtung **100** gemäß Figur 3, welche über eine Verbindungseinheit **101** mit einem Roboter **200** verbunden ist. Der Sensor **106** kann über den Roboter **200** ausgelesen werden, da der Sensor mithilfe der Verbindungseinheit **101** operativ mit dem Roboter verbunden ist.

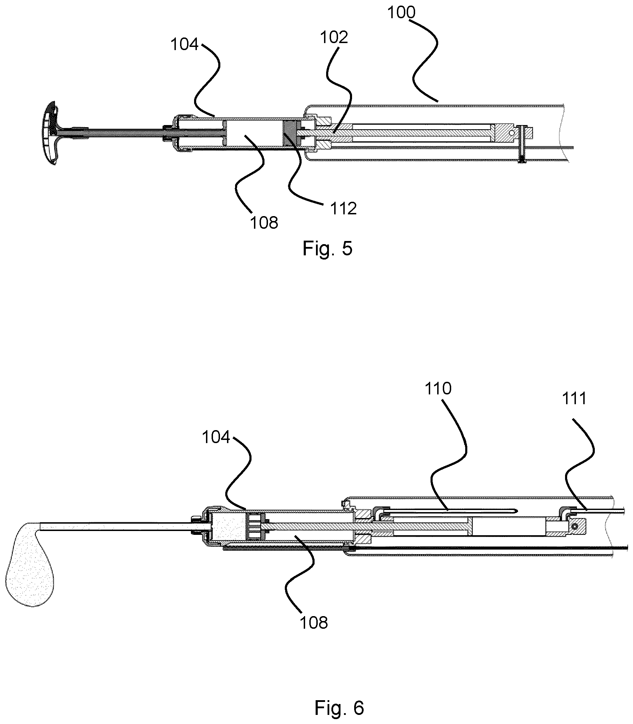

**Fig. 5** zeigt eine weitere Ausführungsform einer erfindungsgemäßen Vorrichtung **100,** welche in diesem Beispiel einen Elektromotor als Antrieb aufweist. Die Vorrichtung **100** ist mit einer Knochenzement-haltigen Kartusche **104** verbunden, welche in diesem Beispiel auch einen entfernbaren Mischstab aufweist, welcher eine manuelle Mischung von Mehrkomponenten-Knochenzement erlaubt. Der Knochenzement ist in einem inneren Volumen **108** angeordnet. Die Vorrichtung umfasst eine Auspresseinheit **102** mit einem Teller **112** zum Ausbringen des Knochenzements.
**Fig. 6** zeigt eine weitere Ausführungsform einer erfindungsgemäßen Vorrichtung **100,** welche in diesem Beispiel einen Druckluftanschluss als Antrieb aufweist, der einen Eingang **110** und einen Ausgang **111** aufweist. Mithilfe des Antriebs kann die Auspresseinheit **102** den Knochenzement aus dem inneren Volumen **108** des Behälters **104** nach außen abgeben, wobei der Behälter hierzu weiterhin einen Schnorchel zum Ausbringen des Knochenzements aufweist.

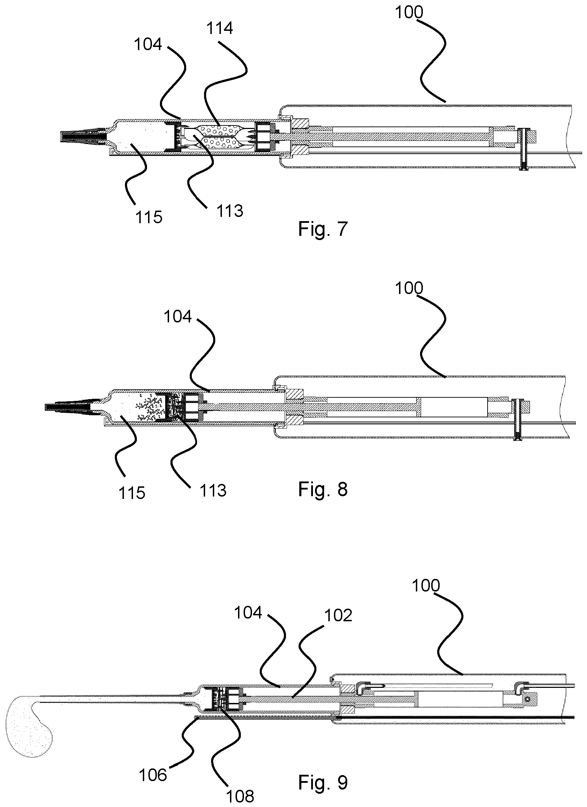

**Fig. 7** zeigt eine weitere Ausführungsform einer erfindungsgemäßen Vorrichtung **100,** welche zum maschinellen Mischen einer flüssigen und einer festen Komponente eines Mehrkomponenten-Knochenzements eingerichtet ist. Die Vorrichtung **100** ist hier mit einem Behälter **104** verbunden, der eine Kammer **113** zur Aufnahme einer flüssigen Komponente und einer Kammer **115** zur Aufnahme einer Pulverkomponente aufweist. Die flüssige Komponente ist in diesem Beispiel in mehreren Folienbeuteln **114** in der Kammer **113** angeordnet. Ein solcher Behälter wird hierin auch als "Full-Prepacked-Mischsystem" bezeichnet.
**Fig. 8** zeigt den Mischvorgang der beiden Knochenzement-Komponenten in einer Vorrichtung **100** gemäß Figur 7. Die Auspresseinheit weist mehrere Dornen auf, um die Folienbeutel **114,** welche die flüssige Komponente enthalten, zu durchstechen. Durch Ausübung von Druck auf die Kammer **113** werden zunächst die Folienbeutel durchstochen, und die austretende Flüssigkeit wird aus der Kammer **113** in die Kammer **115** für die Pulverkomponente gedrückt, sodass sich die beiden Komponenten des Knochenzements mischen und miteinander verbinden können. Der hier beschriebene Mischvorgang kann durch einen OP-Roboter gesteuert werden. In der hier gezeigten Ausführungsform umfasst die Antriebseinheit einen Elektromotor.
**Fig. 9** zeigt eine weitere Ausführungsform einer erfindungsgemäßen Vorrichtung **100,** welche zum maschinellen Mischen einer flüssigen und einer festen Komponente eines Mehrkomponenten-Knochenzements eingerichtet ist. In diesem Beispiel ist die Vorrichtung mit einem Anschluss für ein druckbeaufschlagtes Medium, z.B. Druckluft, ausgestattet. Der mithilfe der Vorrichtung maschinell gemischte Knochenzement kann aus dem inneren Volumen **108** mithilfe eines Schnorchel ausgebracht werden. Die ausgebrachte Menge an Knochenzement wird mithilfe einer Sensorleiste überwacht. Ein mit der Vorrichtung **100** operativ verbundener Roboter **200** steuert anhand eines Signals des Sensors **106** die Auspresseinheit **102,** um eine vorbestimmte Menge Knochenzement abzugeben.

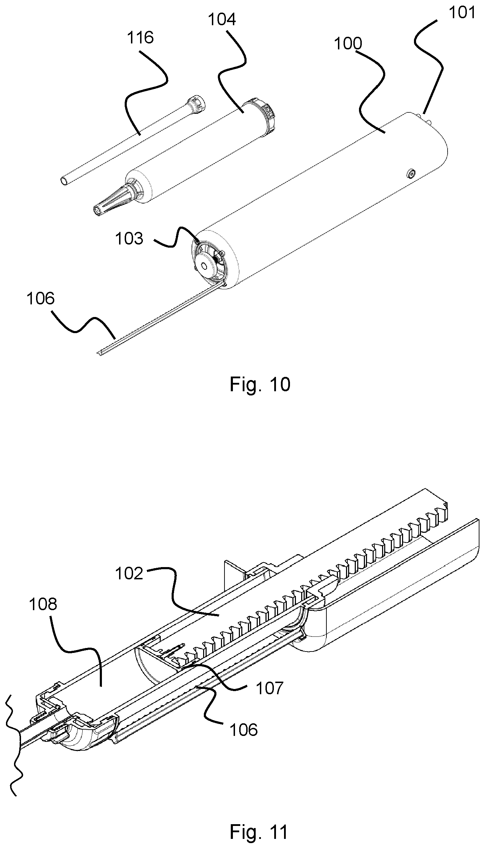

**Fig. 10** zeigt Bestandteile eines Kits, welche eine erfindungsgemäße Vorrichtung **100** mit einer Verbindungseinheit **101,** eine Aufnahmeeinheit **103** und einem Sensor umfasst. Das Kit enthält weiterhin einen Behälter **104** mit Knochenzement, der über die Aufnahmeeinheit **103** mit der Vorrichtung **100** verbunden werden kann. Das Kit enthält optional weiterhin einen Schnorchel **116** bum Ausbringen von Knochenzement, welcher an den Behälter **104** angebracht werden kann.
**Fig. 11** zeigt einen Querschnitt durch eine Ausführungsform einer erfindungsgemäßen Vorrichtung **100,** bei der die Auspresseinheit **102** eine Markierung **107** aufweist, die in der hier gezeigten Ausführung als farblich abgesetzter Dichtungsring ausgestaltet ist. Die Position des Dichtungsrings kann mithilfe einer stabförmigen optischen Sensorleiste **106,** die an der Außenseite der Vorrichtung angeordnet ist, bestimmt werden.

### BEZUGSZEICHENLISTE

- 100: Vorrichtung
- 101: Verbindungseinheit
- 102: Auspresseinheit
- 103: Aufnahmeeinheit
- 104: Behälter
- 105: Schnittstelle
- 106: Sensor
- 107: Markierung
- 108: inneres Volumen
- 109: Antrieb
- 110: Eingang
- 111: Ausgang
- 112: Teller
- 113: Kammer für flüssige Komponente
- 114: Beutel
- 115: Kammer für Pulverkomponente
- 116: Schnorchel
- 117: Griff
- 200: OP-Roboter

## Patentansprüche

1. Vorrichtung (100) zur maschinellen Abgabe von Knochenzement, aufweisend eine Verbindungseinheit (101), die zur Verbindung mit einem chirurgischen Roboter eingerichtet ist, eine Auspresseinheit (102), die zur Abgabe von Knochenzement eingerichtet ist, eine Aufnahmeeinheit (103), die zur Aufnahme eines Behälters (104) mit Knochenzement eingerichtet ist, und eine Schnittstelle (105), die zur Verbindung mit einer Steuereinheit eingerichtet ist, um mit Hilfe der Auspresseinheit (102) eine vorbestimmte Menge Knochenzement aus dem Behälter (104) abzugeben, **dadurch gekennzeichnet, dass** die Vorrichtung einen Sensor (106) aufweist, welcher operativ mit der Schnittstelle (105) verbunden ist, um die Menge des mithilfe der Auspresseinheit (102) abgegebenen Knochenzements zu bestimmen.

2. Vorrichtung nach Anspruch 1, wobei die Schnittstelle (105) eingerichtet ist, die Menge des mithilfe der Auspresseinheit (102) abgegebenen Knochenzements zu übermitteln und bei Erreichen der vorbestimmten Menge eine weitere Abgabe des Knochenzements zu stoppen.

3. Vorrichtung nach Anspruch 1 oder 2, wobei der Sensor (106) ein optischer Sensor, ein mechanischer Sensor oder ein magnetischer Sensor ist.

4. Vorrichtung nach einem der vorangegangenen Ansprüche, wobei der Sensor (106) eingerichtet ist, eine räumliche Position einer Markierung (107) zu detektieren, um dadurch die Menge des mithilfe der Auspresseinheit (102) abgegebenen Knochenzements zu bestimmen, wobei die Markierung bevorzugt eine farbiges Bauteil der Auspresseinheit (102), besonders bevorzugt ein Dichtungsring eines Kolbens, ist.

5. Vorrichtung nach einem der vorangegangenen Ansprüche, wobei der Sensor (106) und/oder die Schnittstelle (105) eingerichtet ist/sind, ein inneres Volumen (108) der Auspresseinheit (102) und/oder des Behälters (104) zu ermitteln.

6. Vorrichtung nach einem der vorangegangenen Ansprüche, wobei der Sensor (106) auf einer Sensorleiste (109) angeordnet ist, die sich auf einer Außenseite der Vorrichtung entlang der Aufnahmeeinheit (103) erstreckt.

7. Vorrichtung nach einem der vorangegangenen Ansprüche, wobei die Verbindungseinheit (101) einen elektrischen Anschluss aufweist, welcher zur Energieversorgung der Vorrichtung und/oder zum Empfang eines Steuersignals eingerichtet ist.

8. Vorrichtung nach einem der vorangegangenen Ansprüche, wobei die Verbindungseinheit (101) einen Anschluss zur Verbindung mit einem druckbeaufschlagten Medium aufweist, wobei das druckbeaufschlagte Medium bevorzugt Druckluft, eine Vakuumquelle oder ein hydraulisches Medium ist.

9. Vorrichtung nach einem der vorangegangenen Ansprüche, wobei die Auspresseinheit (102) einen Antrieb (109) aufweist, der zur Abgabe von Knochenzement mit Hilfe der Auspresseinheit eingerichtet ist.

10. Vorrichtung nach Anspruch 9, wobei der Antrieb (109) ein hydraulischer, pneumatischer oder elektrischer Antrieb ist.

11. Vorrichtung nach Anspruch 9 oder 10, wobei die Schnittstelle (105) zur Steuerung des Antriebs (109) eingerichtet ist.

12. Vorrichtung nach einem der vorangegangenen Ansprüche, wobei die Vorrichtung weiterhin zum maschinellen Mischen von Knochenzement in dem Behälter (104) eingerichtet ist.

13. Computerprogramm zur Steuerung der Vorrichtung nach einem der vorangegangenen Ansprüche, wobei das Computerprogramm zum Betrieb der Auspresseinheit (102), und/oder des Sensors (106) eingerichtet ist, wobei das Computerprogramm zur Durchführung der nachfolgenden Schritte eingerichtet ist:
1) Aktivieren der Auspresseinheit, wobei durch Aktivieren der Auspresseinheit die Abgabe von Knochenzement mithilfe der Vorrichtung erfolgt;
2) Berechnung der Menge des mithilfe der Vorrichtung abgegebenen Knochenzements, basierend auf einem Messwert eines Sensors der Vorrichtung
3) Vergleich dieser Menge mit einem vorbestimmten Wert;
4) Deaktivieren der Auspresseinheit, wenn die Menge den vorbestimmten Wert erreicht oder überschreitet.

14. Kit, aufweisend eine Vorrichtung nach einem der Ansprüche 1 bis 12, und einen Behälter (104) mit Knochenzement, wobei der Behälter bevorzugt zum Mischen von Knochenzement eingerichtet ist.

## Claims

1. A device (100) for dispensing bone cement by means of a machine, comprising a connection unit (101) configured for connection to a surgical robot, a press-out unit (102) configured for dispensing bone cement, a receiving unit (103) configured for receiving a container (104) containing bone cement, and an interface (105) configured for connection to a control unit, in order to dispense a predefined amount of bone cement from the container (104) by means of the press-out unit (102), **characterized in that** the device comprises a sensor (106) which is operatively connected to the interface (105) in order to determine the amount of bone cement dispensed by means of the press-out unit (102).

2. The device according to claim 1, wherein the interface (105) is configured to convey the amount of bone cement dispensed by means of the press-out unit (102) and to stop further dispensing of the bone cement when the predefined amount is reached.

3. The device according to claim 1 or claim 2, wherein the sensor (106) is an optical sensor, a mechanical sensor or a magnetic sensor.

4. The device according to any of the preceding claims, wherein the sensor (106) is configured to detect a spatial position of a marking (107) in order to thereby determine the amount of bone cement dispensed by means of the press-out unit (102), wherein the marking is preferably a colored component of the press-out unit (102), particularly preferably a sealing ring of a plunger.

5. The device according to any of the preceding claims, wherein the sensor (106) and/or the interface (105) is/are configured to ascertain an inner volume (108) of the press-out unit (102) and/or of the container (104).

6. The device according to any of the preceding claims, wherein the sensor (106) is arranged on a sensor strip (109) which extends along the receiving unit (103) on the outside of the device.

7. The device according to any of the preceding claims, wherein the connection unit (101) comprises an electrical connection point which is configured to supply power to the device and/or to receive a control signal.

8. The device according to any of the preceding claims, wherein the connection unit (101) comprises a connection point for connection to a pressurized medium, wherein the pressurized medium is preferably compressed air, a vacuum source or a hydraulic medium.

9. The device according to any of the preceding claims, wherein the press-out unit (102) comprises a drive (109) which is configured for dispensing bone cement by means of the press-out unit.

10. The device according to claim 9, wherein the drive (109) is a hydraulic, pneumatic or electrical drive.

11. The device according to claim 9 or claim 10, wherein the interface (105) is configured to control the drive (109).

12. The device according to any of the preceding claims, wherein the device is also configured for mixing bone cement by means of a machine in the container (104).

13. A computer program for controlling the device according to any of the preceding claims, wherein the computer program is configured to operate the press-out unit (102) and/or the sensor (106), wherein the computer program is configured to carry out the following steps:
1) activating the press-out unit, wherein the dispensing of bone cement by means of the device occurs as a result of activating the press-out unit;
2) calculating the amount of bone cement dispensed by means of the device, on the basis of a measured value from a sensor of the device;
3) comparing this amount with a predefined value;
4) deactivating the press-out unit if the amount reaches or exceeds the predefined value.

14. A kit comprising a device according to any of claims 1 to 12 and a container (104) containing bone cement, wherein the container is preferably configured for mixing bone cement.

## Revendications

1. Dispositif (100) pour la distribution mécanique de ciment osseux, présentant une unité de connexion (101) configurée pour être connectée à un robot chirurgical, une unité d'éjection (102) conçue pour distribuer du ciment osseux, une unité de réception (103) conçue pour recevoir un récipient (104) comportant du ciment osseux, et une interface (105) configurée pour être connectée à une unité de commande afin de distribuer une quantité prédéterminée de ciment osseux provenant du récipient (104) à l'aide de l'unité d'éjection (102), **caractérisé en ce que** le dispositif présente un capteur (106) qui est connecté de manière opérationnelle à l'interface (105) afin de déterminer la quantité de ciment osseux distribué à l'aide de l'unité d'éjection (102).

2. Dispositif selon la revendication 1, dans lequel l'interface (105) est configurée pour transmettre la quantité de ciment osseux distribué à l'aide de l'unité d'éjection (102) et pour arrêter une distribution supplémentaire du ciment osseux lorsque la quantité prédéterminée est atteinte.

3. Dispositif selon la revendication 1 ou 2, dans lequel le capteur (106) est un capteur optique, un capteur mécanique ou un capteur magnétique.

4. Dispositif selon l'une des revendications précédentes, dans lequel le capteur (106) est configuré pour détecter une position spatiale d'un marquage (107) afin de déterminer ainsi la quantité de ciment osseux distribué à l'aide de l'unité d'éjection (102), dans lequel le marquage est de préférence un composant coloré de l'unité d'éjection (102), de manière particulièrement préférée une bague d'étanchéité d'un piston.

5. Dispositif selon l'une des revendications précédentes, dans lequel le capteur (106) et/ou l'interface (105) sont configurés pour estimer un volume interne (108) de l'unité d'éjection (102) et/ou du récipient (104).

6. Dispositif selon l'une des revendications précédentes, dans lequel le capteur (106) est disposé sur une baguette pour capteur (109) s'étendant sur une face externe du dispositif le long de l'unité de réception (103).

7. Dispositif selon l'une des revendications précédentes, dans lequel l'unité de connexion (101) présente une connexion électrique configurée pour alimenter en énergie le dispositif et/ou pour recevoir un signal de commande.

8. Dispositif selon l'une des revendications précédentes, dans lequel l'unité de connexion (101) présente un raccordement pour le raccordement à un milieu sous pression, dans lequel le milieu sous pression est de préférence de l'air comprimé, une source de vide ou un milieu hydraulique.

9. Dispositif selon l'une des revendications précédentes, dans lequel l'unité d'éjection (102) présente un entraînement (109) conçu pour distribuer du ciment osseux à l'aide de l'unité d'éjection.

10. Dispositif selon la revendication 9, dans lequel l'entraînement (109) est un entraînement hydraulique, pneumatique ou électrique.

11. Dispositif selon la revendication 9 ou 10, dans lequel l'interface (105) est configurée pour commander l'entraînement (109).

12. Dispositif selon l'une des revendications précédentes, dans lequel le dispositif est en outre conçu pour mélanger mécaniquement du ciment osseux dans le récipient (104).

13. Programme informatique permettant la commande du dispositif selon l'une des revendications précédentes, dans lequel le programme informatique est configuré pour faire fonctionner l'unité d'éjection (102), et/ou le capteur (106), dans lequel le programme informatique est configuré pour exécuter les étapes suivantes :
1) activation de l'unité d'éjection, dans lequel, en activant l'unité d'éjection, la distribution de ciment osseux est effectuée à l'aide du dispositif ;
2) calcul de la quantité de ciment osseux distribué à l'aide du dispositif, sur la base d'une valeur mesurée par un capteur du dispositif ;
3) comparaison de ladite quantité avec une valeur prédéterminée ;
4) désactivation de l'unité d'éjection lorsque la quantité atteint ou dépasse la valeur prédéterminée.

14. Kit, présentant un dispositif selon l'une des revendications 1 à 12, et un récipient (104) comportant du ciment osseux, dans lequel le récipient est de préférence conçu pour mélanger le ciment osseux.
